# EUROPEAN PATENT SPECIFICATION

(11) **EP 4 374 383 B1**
(45) Date of publication and mention of the grant of the patent: **03.09.2025**
(21) Application number: 22751068.2
(22) Date of filing: 15.07.2022
(51) Int. Cl.: G16H 20/30, G16H 40/67, G10H 1/42

(54) **MUSIC BASED EXERCISE PROGRAM**
MUSIKBASIERTES TRAININGSPROGRAMM
PROGRAMME D'EXERCICE BASÉ SUR LA MUSIQUE

(30) Priority: 19.07.2021 EP 21186370
(43) Date of publication of application: 29.05.2024
(73) Proprietor: Intelligent Training Group APS, 4180 Sorø (DK)
(72) Inventor: MØLLER HANSEN, Peter, 4180 Sorø (DK); OVERKÆR, Brian, 4180 Sorø (DK); WILLEMOES HANSEN, Anders, 4180 Sorø (DK)
(74) Representative: Budde Schou A/S
(86) International application number: PCT/EP2022/069878
(87) International publication number: WO 2023/001710

(56) References cited:
- US-A1- 2008 134 862
- US-A1- 2015 081 066
- US-A1- 2018 005 615

## Description

The present invention generally relates to generating an exercise program comprising a plurality of exercise intervals, wherein the generation of the exercise program is based on music e.g., in a playlist.

### BACKGROUND

Athletic activities are often performed while listening to music, where the music often functions as a motivator for the athlete. High intensity athletic activities are often performed while listening to intense music e.g., having a fast rhythm or where the music in some other way is more intense, whereas low intensity athletic activities are accompanied by slower music having a low beat or in some other way is less intense. Thereby the athlete can use the music as a training support and motivation factor encouraging the athlete to adjust activity to the music and perform accordingly.

Typically, when training, the athlete trains in exercise intervals, where the entire exercise program could comprise a number of exercise intervals and an example of a simple exercise program could be as follows: first a warmup exercise interval with a low intensity, then an exercise interval where the intensity is higher, then an exercise interval where the intensity is very high and finally a cool down exercise interval with a low intensity pace recovery.

If the athlete wishes to accompany such exercise program with music, then ideally a playlist with music needs to be built where both intensity (e.g. rhythm), timing (length of music piece with the desired rhythm) corresponds to the exercise intervals and the music taste of the athlete.

In US7795523B2 and US108787192, playlists are created to fit the intensity of exercise programs. In US7795523B2, an exercise program is created, and records of songs used for previous intensities (energy consumption) are then used for, based on link between intensity and previous selected music, generating playlists for the exercise program. Also, in US108787192, playlists are suggested to fit the heartrate/intensity correlated with the music at previous exercise routines. Playlists may be e.g., selected based on speed or pace (US10878719B2).

A problem with the above scenario is that playlists must be fitted to an exercise program and in order to make a fit, it can be necessary to compromise between the selected songs to make a good fit to the exercise program. Using songs for an exercise program based on the athletes energy consumption on previous exercise is also somewhat random, since natural challenges (e.g. terrain and wind) could increase energy consumption even when listening to music with low energy. Further, these concepts do not take the athletes own motivation for music in consideration and is purely based on simple music analysis without reference to what style of music the athletes are motivated by when exercising. Using a song for a predefined workout does not allow for exercise intervals based on the music sections within each song and is therefore only creating simply exercise programs with each song being an "exercise intensity" for the preselected workout, which is less engaging compared to using in depth audio analysis for each song to match exercise intervals. It is also known that that this scanning of songs to generate a playlist that fits can be very time consuming and requires much processing power of the computer system which could be a smart phone.

It is an object of the present invention to solve some of the above-mentioned problems.

### SUMMARY OF THE INVENTION

This is obtained by a computer-implemented method for generating an exercise program comprising a plurality of exercise intervals, the method comprising the consecutive steps of:
- providing a playlist comprising at least one music track,
- analysing said at least one music track in said playlist to identify a plurality of music sections, wherein each interval is identified based on identification of musical characteristics such as musical elements and time flow,
- generating an exercise program, said exercise program comprising a plurality of exercise intervals wherein at least the timing and intensity of each exercise interval corresponds to one or more consecutive identified music sections of said at least one music track in said playlist.

Thereby, the user, e.g., the athlete or an instructor, can end up with an exercise program where there is a fit between the exercise intervals in the exercise program and the music accompanying the training program. An intuitive feel of intensity is obtained based on the songs in the playlist accompanying the training program, because the exercise program and its exercise intervals have been generated based on the music and the music sections in the music. Further, the user does not have to come up with an exercise program of their own. They need to provide a playlist (list of songs) and the method will generate an exercise program according to the playlist. This method is a very fast way to generate an exercise program with a motivating playlist, basically a playlist is necessary for generating the exercise program, but in an embodiment, some preferences regarding the exercise could also be fed to a computer by the athlete and based on this input, an exercise program is made, which corresponds to the playlist. When generating the exercise programs and its exercise intervals based on the identified music sections in the music, it means that the timing (start and stop of exercise interval), the duration of the exercise interval and the intensity of the exercise interval are generated so that they will correspond to the identified music sections, whereby the timing of the exercise interval corresponds to the timing of the identified music interval (the exercise interval should start when a music interval starts and stop when a music interval stops), the duration of the exercise interval should be the same as one or more consecutive music sections, and the exercise intensity of the exercise interval should correspond to the music intensity of the music interval.

In an embodiment, said step of identifying a plurality of music sections comprises reading metadata attached to said music track and identifying said music sections based on said metadata. By using metadata already attached to the music, it might not be necessary to perform an actual analysis of the music by analysis software, thereby obtaining musical characteristics is not very demanding for the computer system.

In an embodiment, said step of identifying a plurality of music sections comprises analyzing said at least one music track and identifying said music sections based on said analysis. Thereby, an analysis can be set up to identify music sections using dedicated music analysis software, where based on settings in the software it is possible to identify music sections. Typically, the analysis would comprise identifying musical elements throughout the music and then grouping these to identify music sections with similar characteristics.

In an embodiment, musical characteristics comprise one or more of the following music characteristics data: Song title, artist title, beat, meter, dynamics, harmony, melody, pitch, rhythm, tempo, texture, timbre, intro, verse, chorus, bridge. These characteristics are data that have proven good for dividing/grouping music into music sections.

In an embodiment, a musical interval is defined and identified as an interval having at least one musical characteristic being substantially identical during the entire musical interval.

In an embodiment, an exercise interval corresponds to at least two consecutive identified musical sections. Thereby, the exercise interval is not locked to having the same length as music sections, since music sections can be quite short compared to the length of desired exercise intervals. In general, an exercise interval should end at the end of a music interval, but the duration of an exercise interval might be several music sections.

In an embodiment, an exercise interval in said exercise program is generated with an intensity based on the intensity of said music. Intensity similarity between music and exercise has proven to be a good and motivating match. Generally, intensity of music is measured relative to other music sections in the same music, where an intense music section is more intense than other music sections in the music. A heavy metal track might be intense during the entire track and much more intense than a soft pop track, but sections in the heavy metal track still have different relative intensity, which is used to divide the track into music sections with different intensity.

In an embodiment, high intensity music interval results in high intensity exercise interval.

In an embodiment, the step of providing a playlist comprises defining said playlist by combining a number of music tracks in a desired order.

In an embodiment, the step of providing a playlist comprises selecting said playlist from a list of predefined playlists.

In an embodiment the identification of a plurality of music sections in said at least one music track is further based on historic data linking music previously selected by users in relation to their exercise programs. Thereby identifying music sections in music tracks is further based on looking at previous users usage of that specific music track if e.g. a significant number of users use a music section in the music track for high intensity training then this sections could be identified as a music section and as a music section suitable for high intensity exercise intervals - similarly if a significant number of users use a music section in the music track for low intensity training then this sections could be identified as a music section and as a music section suitable for low intensity exercise intervals. When generating the exercise program this knowledge could be used in combination with the identification of musical characteristics of the music track to generate an exercise program with a plurality of exercise intervals, wherein at least the timing and intensity of each exercise interval correspond to one or more consecutive identified music sections of said at least one music track in said playlist. The historic data could be stored in a database collecting links between music and exercise programs and as the amount of data increases the probability that the music sections is suitable for specific types of training intervals increases.

The invention further relates to a system for generating an exercise program comprising a plurality of exercise intervals and a machine-readable storage medium containing one or more programs for generating an exercise program according to the above.

### SUMMARY OF FIGURES

Figure 1 illustrates the principle of the present invention,
Figure 2 illustrates an exercise program generated based one or more music tracks, e.g., in a playlist,
Figure 3 illustrates a method of generating an exercise program based on a playlist,
Figure 4 illustrates a system for generating an exercise program based on a playlist.

### DETAILED DESCRIPTION

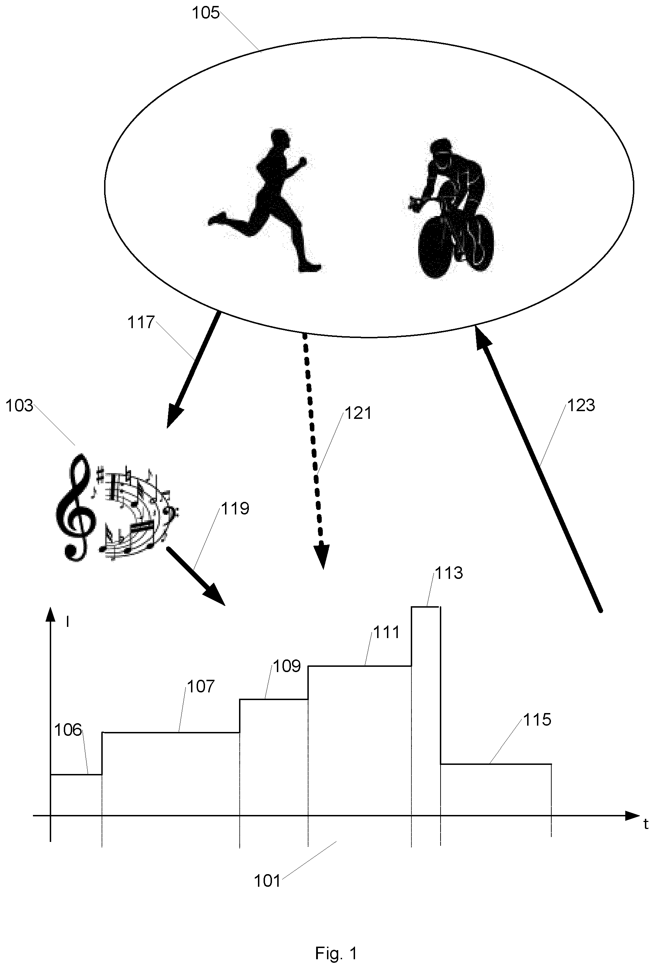

Figure 1 illustrates the principle of the invention, which generally relates to a method of generating an exercise program based on one or more music tracks e.g. in a playlist.

It is of interest to end up with an exercise program 101 and a playlist 103 with music that can be listened to during the exercise program by the athlete 105 and to ensure that the exercise intervals (106, 107, 109, 111, 113, 115) of the exercise program fits to one or more consecutive music sections of the music in the playlist.

The athlete 105 can, as illustrated by the arrow 117, initially choose a list of music tracks 103 that have already been generated or a list can be created specifically to the activity by the athlete. Next, as illustrated by the arrow 119, the music is used as basis for generating a corresponding exercise program 101. The exercise program may be created solely based on the music 103, but as an alternative and illustrated by the dotted arrow 121, the athlete might have some specific wishes relating to the exercise program (such as length of exercise program, suggestions to reorder the list of music tracks, limitations to intensity in exercise program, alternative music tracks to be added to the list of music, etc.). These wishes could be embedded in the settings of the exercise program generation algorithm, or they could be obtained by prompting the athlete during creation of the exercise program.

Finally, and as illustrated by the arrow 123, the exercise program is used by the athlete, accompanied by music tracks, where exercise intervals of the exercise program (intensity and duration) match music sections of the music.

An interval in the exercise program could be defined by exercise intensity, e.g. Pulse/Heart Rate, Watt, FTP, %FTP, Position, Speed, Distance Traveled, MAP, %MAP, FTHR, %FTHR, Power Zones, LTHR, %LTHR, VO2max, %VO2max, RPE Levels, Kcal, Strokes, Time (time will affect the RPE levels). This is just examples of data that can determine intensity in an exercise program and it will depend on the exercise activity, which could be anything from cycling, running, skiing or rowing where it is possible to train with different intensities or any other exercise where intervals are used for training.

The exercise program illustrated in 101 has several exercise intervals 106, 107, 109, 111, 115, where the intensity (I) varies over time (t) from interval to interval where e.g., interval 106, and 105 has the lowest intensities and interval 113 has the highest intensity. Further, also the duration of each interval varies, where interval 107 is the longest interval and interval 113 is the shortest and is a result of the music analysis.

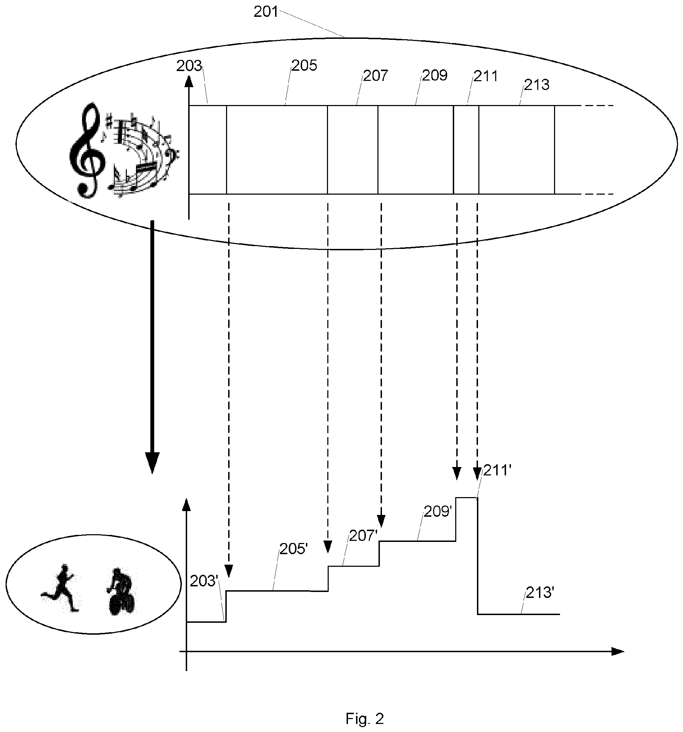

In figure 2, the generation of an exercise program based on music tracks is illustrated and explained in more detail. The music is divided into music sections based on a music analysis and characteristics of the music and then exercise intervals are generated, which correspond to the music sections. Typically, the music comprises many music sections and each music interval will not necessarily correspond to one exercise interval, instead consecutive music sections are grouped to correspond to one exercise interval where timing, duration and intensity of the group of music sections correspond to timing, duration and intensity of one exercise interval.

Some music characteristics that could be identified to define music sections could be characteristics defining the musical sound such as rhythm/beat, base, sound level, dynamics, harmony, pitch, timbre. See a more detailed definition of characteristics of musical elements in the below table:

| | | |
|---|---|---|
| Element | Definition | Characteristics |
| **Beat** | Gives music its rhythmic | A beat can be regular or irregular. pattern |
| **Meter** | Rhythmic patterns produced by grouping together strong weak beats | A meter may be two or more beats in a and measure. |
| **Dynamics** | The volume of a performance | Like punctuation marks, dynamics abbreviations and symbols indicate moments of emphasis. |
| **Harmony** | The sound producted when two or more notes are played at the same time | Harmony supports the melody and gives it texture. |
| **Melody** | The oevrarching tune created by playing a succession or series of notes | A composition may have a single or multiple melodies. |
| **Pitch** | A sound based on the frequency of vibration and size of the vibrating objects | The slower the vibration and the bigger the vibrating object, the lower the pitch will be a nd vice versa. |
| **Rhythm** | The pattern or placement of sounds in time and beats in music | Rhythm is shaped by meter and has elements such as beat and tempo. |
| **Tempo** | The speed at which a piece of music is played | The tempo is indicated by an Italian word at the beginning of a score, such as a *largo* for slow or *presto* for very fast. |
| **Texture** | The number and types of layers used in a composition | A texture may be a single line, two or more lines, or the main melody accompanied by chords. |
| **Timbre** | The quality of the sound that distinguishes one voice or instrument from another | Timbre can range from dull to lush |

Beside musical elements for identifying possible musical sections in each piece of music, the time flow of each piece of music is also analyzed, this includes identifying elements such as intro, verse, chorus, bridge etc. Typically, the verse describes the concept of the title and hook that are typically in the chorus. Other music sections will function to support these main components of the song. Music sections could consist of measures (also called bars) that are typically four beats in length. Although they can be longer or shorter, music sections are typically eight measures (bars) in length.

The above are just examples of musical elements that can be used to divide the music into music sections, there will be different methodologies of identifying music sections and thereby use these music sections for generating exercise intervals.

Some musical information might in one embodiment be part of the music file, where it could be attached as meta data and more or all information could also be obtained from music analysis tools such as Spotify Audio Analysis, Musicscope or similar software.

When the musical characteristics are identified, then music sections can be determined based on the characteristics and from these music sections, an exercise program can be generated where exercise intervals fit one or more consecutive music sections both in timing, duration and in intensity.

Another method to identify possible musical sections in a specific piece of music could be based on stored data relating to previous users exercise program during the piece of music. By having data linking music with previous users exercise programs, it is possible to identify links between music and previous exercise and thereby sections could be identified as typically used for:
- interval start,
- interval end,
- high intensity training,
- the middle of an exercise program,
- etc.

These data will be improved as the number of users increases.

Music sections could be identified solely by analysing the musical characteristics or solely based on previous users or by combining the two methods.

In the example of figure 2, the start of the exercise program is generated having exercise intervals 203', 205', 207', 209', 211', 213' and these are generated based on the first part of the music 201 being divided into the musical sections 203, 205, 207, 209, 211, 213.

The exercise program has been generated where the first exercise interval 203' is a low intensity exercise interval and the duration of this corresponds to the music interval 203. This could be a music interval which is the intro, which it has a low music intensity. Then in 205, the music gets a higher intensity, e.g. drums start playing, or another musical change happens which is a good timing for changing the exercise intensity to a higher intensity in the next exercise interval 205'. Then in 207', the intensity is increased again and the timing of this is the result of a new music interval 207 where the music e.g., reaches another phase. Then in 209, the music gets an even higher intensity, which is a good timing for changing the exercise intensity to an even higher intensity in the next exercise interval 209'. In 211, the music could reach its chorus, and this could be a good time to reach maximum intensity in the exercise interval 211' and when the chorus ends in 213', a very low intensity exercise interval is initiated to recover. The exercise could continue in a similar manner, where the exercise intervals of the exercise program constantly change according to one or more consecutive music sections of the music and the exercise intervals of the exercise program could continue in a similar manner, where loops in a music piece could result in similar timed loops in the exercise program.

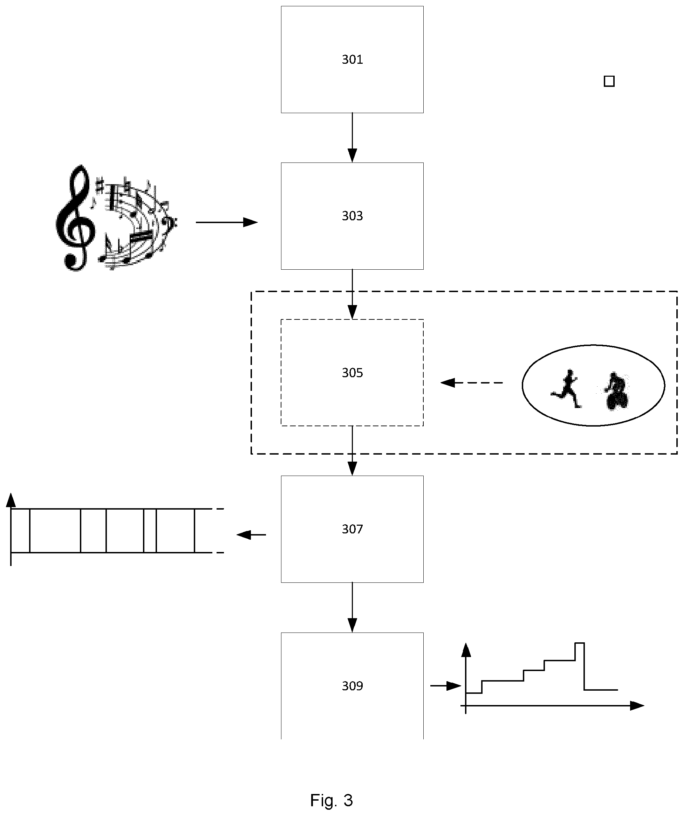

In figure 3, the method of the present invention is illustrated using a flow diagram, the method being typically performed by a computer running software implementing the method.

In 301, the software program is started, e.g. by starting the program, e.g. on a laptop or stationary computer or it could be started on a mobile device such as a smartphone or similar. When the software has been initiated, then a user screen is presented to the athlete enabling the athlete to make further selections using a keyboard, mouse, or a touch screen. The athlete could then choose to generate a new exercise program based on music tracks and the athlete is prompted to either select an already predefined playlist identifying a number of music tracks or a playlist could be generated at this stage by the athlete. The predefined playlists could be loaded from the private music collection of the athlete or from an on-demand music service.

In 303, a playlist has been selected or made and the length of this playlist could in one embodiment correspond to the length of the exercise program to be generated. The data of this playlist is loaded for generating an exercise program based on the playlist.

Now, the music on which the exercise program should be based has been selected and the generation of the exercise program using the software algorithm could be made solely based on this playlist not considering any user specific information whereby 305 is skipped and 307 is the next step.

In 305, input from the athlete is received and this input could relate to preferences and data about the athlete and could include data such as:
- performance during previous exercise programs,
- real age, exercise age,
- goal with exercise,
- wishes to intensity,
- wishes to length of exercise intervals,
- specific wishes to the playlist.

These data can be used as a guideline by the software when the exercise program is generated based on the playlist and will overrule the data already present in the software program.

In 307, the musical content of the playlist is analyzed to determine possible music sections and based on this analysis an exercise program is generated in 309. The exercise program is generated, whereby the length and the intensity of the exercise intervals matches the music sections, whereby a music interval is mapped to an exercise interval. The number of generated exercise intervals could be solely based on the playlist and some predefined criteria in the software, or it could be at least partly defined by the user input described in 305. When generating the exercise program, one setting could be that the exercise interval in the exercise program has to be in the same order as the music sections of the playlist, or the software could be allowed by the athlete to rearrange the position of each music track in the playlist to enable a better fit of the generated exercise program with the music allowing the exercise intervals of the exercise program to follow a structured line with a warm up interval, main training interval, final training interval and finally a cool down interval.

Finally, an exercise program is generated, where intensity and exercise intervals are based on the playlist, and where there is a match between timing and intensity between music interval in the playlist and the exercise intervals in the exercise program.

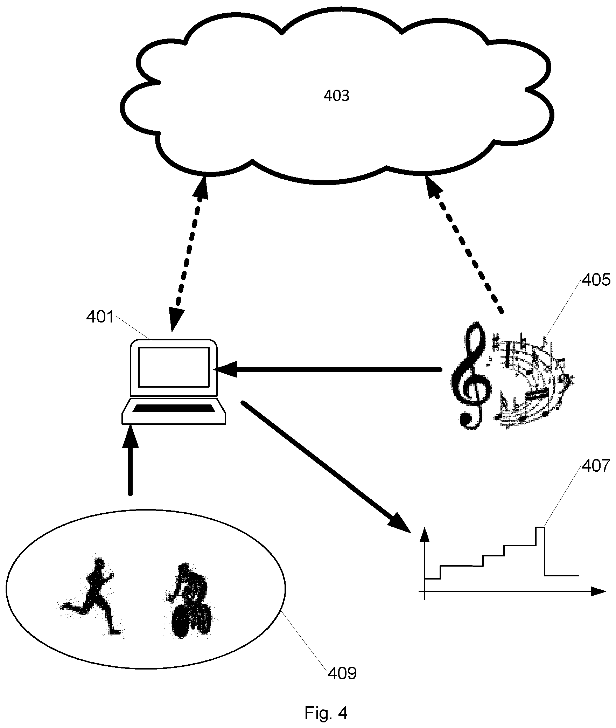

In figure 4, a system for performing a method as described above is illustrated, the system comprising a computer 401 with a display and it could e.g., be a laptop, stationary PC, or a smartphone. The computer is adapted to perform the method of the present invention via a software program for performing the method as described above. The athlete 409 then operates the computer 401 and selects, uploads, or generates the playlist 405 based on which the exercise program 407 is to be generated, the playlist could e.g., be a playlist stored locally in the computer or the computer could be connected to the Internet 403 for accessing data stored in the cloud. Similarly, software for analyzing the musical content of the playlist could be locally on the computer or via a cloud-based service.

## Claims

1. A computer-implemented method for generating an exercise program comprising a plurality of exercise intervals, the method comprising the consecutive steps of:
- providing a playlist comprising at least one music track,
- identifying a plurality of music sections in said at least one music track, wherein each music interval is identified based on identification of musical characteristics such as musical elements and time flow,
- generating an exercise program, said exercise program comprising a plurality of exercise intervals, wherein at least the timing and intensity of each exercise interval correspond to one or more consecutive identified music sections of said at least one music track in said playlist.

2. A method according to claim 1, wherein said step of identifying a plurality of music sections comprises analyzing said at least one music track and identifying said music sections based on said analysis.

3. A method according to claim 1-2, wherein said step of identifying a plurality of music sections comprises reading metadata attached to said music track and identifying said music sections based on said metadata.

4. A method according to claim 1-3, wherein musical characteristics comprise one or more of the following music characteristics; data song title, artist title, beat, meter, dynamics, harmony, melody, pitch, rhythm, tempo, texture, timbre, intro, verse, chorus, bridge.

5. A method according to claim 1-4, wherein a musical section is defined and identified as an interval having at least one musical characteristic being substantially identical during the entire musical section.

6. A method according to claim 1-5, wherein an exercise interval corresponds to at least two consecutive identified musical sections.

7. A method according to claim 1-6, wherein an exercise interval in said exercise program is generated with an intensity based on the intensity of said music.

8. A method according to claim 1-7, wherein high intensity music sections result in high intensity exercise interval.

9. A method according to claim 1-8, wherein the step of providing a playlist comprises defining said playlist by combining a number of music tracks in a desired order.

10. A method according to claim 1-9, wherein the step of providing a playlist comprises selecting said playlist from a list of predefined playlists.

11. A method according to claim 1-10, wherein the identification of a plurality of music sections in said at least one music track is further based on historic data linking music previously selected by users in relation to their exercise programs.

12. A system for generating an exercise program comprising a plurality of exercise intervals, the system comprising:
a memory; and
at least one processor, coupled to the memory, operative to perform the consecutive steps of:
- providing a playlist comprising at least one music track,
- analysing said at least one music track in said playlist to identify a plurality of music sections, wherein each music section is identified based on identification of musical characteristics such as musical elements and time flow,
- generating an exercise program, said exercise program comprising a plurality of exercise intervals wherein at least the timing and intensity of each exercise interval corresponds to one or more consecutive identified music sections of said at least one music track in said playlist.

13. A machine readable storage medium containing one or more programs for performing a method of providing an interactive training environment for athletic activities according to claim 1-11.

## Patentansprüche

1. Computerimplementiertes Verfahren zur Generierung eines Trainingsprogramms, umfassend eine Vielzahl von Trainingsintervallen, wobei das Verfahren die aufeinanderfolgenden Schritte umfasst:
Bereitstellen einer Wiedergabeliste, umfassend wenigstens einen Musiktitel,
Identifizieren einer Vielzahl von Musikabschnitten in dem wenigstens einen Musiktitel, wobei jedes Musikintervall basierend auf der Identifizierung musikalischer Merkmale wie musikalischer Elemente und des Zeitflusses identifiziert wird,
Generieren eines Trainingsprogramms, wobei das Trainingsprogramm eine Vielzahl von Trainingsintervallen umfasst, wobei wenigstens das Timing und die Intensität jedes Trainingsintervalls einem oder mehreren aufeinanderfolgenden identifizierten Musikabschnitten des wenigstens einen Musiktitels in der Wiedergabeliste entsprechen.

2. Verfahren nach Anspruch 1, wobei der Schritt des Identifizierens einer Vielzahl von Musikabschnitten das Analysieren des wenigstens einen Musiktitels und das Identifizieren der Musikabschnitte basierend auf der Analyse umfasst.

3. Verfahren nach Anspruch 1-2, wobei der Schritt des Identifizierens einer Vielzahl von Musikabschnitten das Lesen von Metadaten, die dem Musiktitel beigefügt sind, und das Identifizieren der Musikabschnitte basierend auf den Metadaten umfasst.

4. Verfahren nach Anspruch 1-3, wobei musikalische Merkmale eines oder mehrere der folgenden Musikmerkmale umfassen: Daten-Songtitel, Künstlername, Beat, Takt, Dynamik, Harmonie, Melodie, Tonhöhe, Rhythmus, Tempo, Textur, Klangfarbe, Intro, Strophe, Refrain, Bridge.

5. Verfahren nach Anspruch 1-4, wobei ein Musikabschnitt als ein Intervall definiert und identifiziert wird, das wenigstens ein musikalisches Merkmal aufweist, das während des gesamten Musikabschnitts im Wesentlichen identisch ist.

6. Verfahren nach Anspruch 1-5, wobei ein Trainingsintervall wenigstens zwei aufeinanderfolgenden identifizierten Musikabschnitten entspricht.

7. Verfahren nach Anspruch 1-6, wobei ein Trainingsintervall in dem Trainingsprogramm mit einer Intensität generiert wird, die auf der Intensität der Musik basiert.

8. Verfahren nach Anspruch 1-7, wobei Musikabschnitte mit hoher Intensität zu einem Trainingsintervall mit hoher Intensität führen.

9. Verfahren nach Anspruch 1-8, wobei der Schritt des Bereitstellens einer Wiedergabeliste das Definieren der Wiedergabeliste durch Kombinieren einer Anzahl von Musiktiteln in einer gewünschten Reihenfolge umfasst.

10. Verfahren nach Anspruch 1-9, wobei der Schritt des Bereitstellens einer Wiedergabeliste das Auswählen der Wiedergabeliste aus einer Liste vordefinierter Wiedergabelisten umfasst.

11. Verfahren nach Anspruch 1-10, wobei die Identifizierung einer Vielzahl von Musikabschnitten in dem wenigstens einen Musiktitel ferner auf historischen Daten basiert, die zuvor von Benutzern ausgewählte Musik in Bezug auf deren Trainingsprogramme verknüpfen.

12. System zur Generierung eines Trainingsprogramms, umfassend eine Vielzahl von Trainingsintervallen, wobei das System umfasst:
einen Speicher; und
wenigstens einen Prozessor, der mit dem Speicher gekoppelt und betreibbar ist, um die aufeinanderfolgenden Schritte durchzuführen:
Bereitstellen einer Wiedergabeliste, umfassend wenigstens einen Musiktitel,
Analysieren des wenigstens einen Musiktitels in der Wiedergabeliste, um eine Vielzahl von Musikabschnitten zu identifizieren, wobei jeder Musikabschnitt basierend auf der Identifizierung musikalischer Merkmale wie musikalischer Elemente und des Zeitflusses identifiziert wird,
Generieren eines Trainingsprogramms, wobei das Trainingsprogramm eine Vielzahl von Trainingsintervallen umfasst, wobei wenigstens das Timing und die Intensität jedes Trainingsintervalls einem oder mehreren aufeinanderfolgenden identifizierten Musikabschnitten des wenigstens einen Musiktitels in der Wiedergabeliste entsprechen.

13. Maschinenlesbares Speichermedium, das ein oder mehrere Programme zur Durchführung eines Verfahrens zur Bereitstellung einer interaktiven Trainingsumgebung für sportliche Aktivitäten nach Anspruch 1-11 enthält.

## Revendications

1. Procédé mis en œuvre par ordinateur pour générer un programme d'exercice comprenant une pluralité d'intervalles d'exercice, le procédé comprenant les étapes consécutives suivantes :
- la fourniture d'une liste de lecture comprenant au moins une piste musicale,
- l'identification d'une pluralité de sections musicales dans ladite au moins une piste musicale, dans lequel chaque intervalle musical est identifié sur la base de l'identification de caractéristiques musicales telles que des éléments musicaux et un écoulement du temps,
- la génération d'un programme d'exercice, ledit programme d'exercice comprenant une pluralité d'intervalles d'exercice, dans lequel au moins la synchronisation et l'intensité de chaque intervalle d'exercice correspondent à une ou plusieurs sections musicales identifiées consécutives de ladite au moins une piste musicale dans ladite liste de lecture.

2. Procédé selon la revendication 1, dans lequel ladite étape d'identification d'une pluralité de sections musicales comprend l'analyse de ladite au moins une piste musicale et l'identification desdites sections musicales sur la base de ladite analyse.

3. Procédé selon la revendication 1 et 2, dans lequel ladite étape d'identification d'une pluralité de sections musicales comprend la lecture de métadonnées attachées à ladite piste musicale et l'identification desdites sections musicales sur la base desdites métadonnées.

4. Procédé selon la revendication 1 à 3, dans lequel les caractéristiques musicales comprennent une ou plusieurs des caractéristiques musicales suivantes ; des données de titre de chanson, un titre de l'artiste, un rythme, une mesure, une dynamique, une harmonie, une mélodie, une hauteur, un rythme, un tempo, une texture, un timbre, une intro, un couplet, un refrain, un pont.

5. Procédé selon la revendication 1 à 4, dans lequel une section musicale est définie et identifiée comme un intervalle ayant au moins une caractéristique musicale étant sensiblement identique pendant toute la section musicale.

6. Procédé selon la revendication 1 à 5, dans lequel un intervalle d'exercice correspond à au moins deux sections musicales identifiées consécutives.

7. Procédé selon la revendication 1 à 6, dans lequel un intervalle d'exercice dans ledit programme d'exercice est généré avec une intensité basée sur l'intensité de ladite musique.

8. Procédé selon la revendication 1 à 7, dans lequel des sections musicales de haute intensité donnent lieu à des intervalles d'exercice de haute intensité.

9. Procédé selon la revendication 1 à 8, dans lequel l'étape de fourniture d'une liste de lecture comprend la définition de ladite liste de lecture en combinant un certain nombre de pistes musicales dans un ordre souhaité.

10. Procédé selon la revendication 1 à 9, dans lequel l'étape de fourniture d'une liste de lecture comprend la sélection de ladite liste de lecture à partir d'une liste de listes de lecture prédéfinies.

11. Procédé selon la revendication 1 à 10, dans lequel l'identification d'une pluralité de sections musicales dans ladite au moins une piste musicale est également basée sur des données historiques mettant en lien la musique précédemment sélectionnée par les utilisateurs et leurs programmes d'exercice.

12. Système de génération d'un programme d'exercice comprenant une pluralité d'intervalles d'exercice, le système comprenant :
une mémoire ; et
au moins un processeur, couplé à la mémoire, opérationnel pour réaliser les étapes consécutives de :
- fourniture d'une liste de lecture comprenant au moins une piste musicale,
- d'analyse de ladite au moins une piste musicale dans ladite liste de lecture pour identifier une pluralité de sections musicales, dans lequel chaque section musicale est identifiée sur la base de l'identification de caractéristiques musicales telles que des éléments musicaux et un écoulement du temps,
- de génération d'un programme d'exercice, ledit programme d'exercice comprenant une pluralité d'intervalles d'exercice, dans lequel au moins la synchronisation et l'intensité de chaque intervalle d'exercice correspondent à une ou plusieurs sections musicales identifiées consécutives de ladite au moins une piste musicale dans ladite liste de lecture.

13. Support de stockage lisible par machine contenant un ou plusieurs programmes destinés à réaliser un procédé de fourniture d'un environnement d'entraînement interactif pour des activités sportives selon la revendication 1 à 11.
